# EUROPEAN PATENT APPLICATION

(11) **EP 2 520 231 A1**
(43) Date of publication of application: **07.11.2012**
(21) Application number: 10840956.6
(22) Date of filing: 24.12.2010
(51) Int. Cl.: A61B 17/06

(54) **SUTURE NEEDLE**

(30) Priority: 28.12.2009 JP 2009298915
(71) Applicant: Keisei Medical Industrial Co., Ltd., Bunkyo-ku, Tokyo 113-0033 (JP)
(72) Inventor: UETAKE, Tsuyoshi, Tokyo 113-0033 (JP)
(74) Representative: HOFFMANN EITLE
(86) International application number: PCT/JP2010/073383
(87) International publication number: WO 2011/081100

(57) **Abstract**

The present invention addresses the problem of providing an innovative suture needle which exhibits unprecedented effects. An uneven part is provided to left and right side parts of a distal end site of a needle body (1), and edge parts of the uneven part are configured as small blades (6, 7).

## Description

### TECHNICAL FIELD

The present invention relates to a suture needle to be used in, for example, surgical procedures.

### BACKGROUND ART

With regard to suture needles to be used in, for example, surgical procedures, in order to improve insertability into body tissues, the present applicants have proposed a suture needle in which the cross-sectional shape of a needle body is made into a polygon, as disclosed in Japanese Laid-open Patent Publication 2000-139931, as well as a suture needle in which a recessed groove part is formed in the lengthwise direction positioned to face a peripheral surface of a needle body, as disclosed in Japanese Laid-open Patent Publication 2007-268136.

These suture needles, when inserted into a body tissue, have a smaller surface area of contact with the site of insertion (the body tissue) and are thereby imparted reduced insertion resistance; the body tissue is consequently less likely to be stressed, and favorable suturing work can be performed.
[Patent Document 1] Japanese Laid-open Patent Publication 2000-139931
[Patent Document 2] Japanese Laid-open Patent Publication 2007-268136

### SUMMARY OF THE INVENTION

### PROBLEMS TO BE SOLVED BY THE INVENTION

The present inventors, having labored over further research and experimentation with regard to these suture needles, have developed, as a result thereof, a innovative suture needle which exhibits unprecedented effects.

### MEANS FOR SOLVING THE PROBLEMS

The main points of the present invention shall now be described with reference to the accompanying drawings.

A suture needle according to a first aspect is characterized in that an uneven part is provided to left and right side parts of a distal end site of a needle body 1, and an edge part of the uneven part is configured as a small blade 6, 7.

A suture needle according to a second aspect is the suture needle according to the first aspect, characterized in that the small blade 6, 7 is provided to an edge part of a raised part 4, 5 of the uneven part.

A suture needle according to a third aspect is the suture needle according to the second aspect, characterized in that the raised part 4, 5 is provided in a plurality in a lengthwise direction of the needle body 1.

A suture needle according to a fourth aspect is the suture needle according to any of the first to third aspects, characterized in that the distal end site of the needle body 1 is configured to have a triangular shape in cross-section, and the uneven part is provided to each of left and right side edges 2, 3 of the distal end site having a triangular shape in cross-section.

A suture needle according to a fifth aspect is characterized in that a raised part 4, 5 is provided to left and right side parts of a distal end site of a needle body 1, and an edge part of each of the raised parts 4, 5 on the left and right is configured as a small blade 6, 7.

A suture needle according to a sixth aspect is the suture needle according to the fifth aspect, characterized in that a plurality of each of the raised parts 4, 5 on the left and right is provided.

A suture needle according to a seventh aspect is the suture needle according to the fifth or sixth aspect, characterized in that a plurality of each of the raised parts 4, 5 on the left and right is provided in series along a lengthwise direction of the needle body 1.

A suture needle according to an eighth aspect is the suture needle according to the fifth aspect, characterized in that a rectilinear ridge part 2, 3 extending in the lengthwise direction of the needle body 1 is provided to the left and right side parts of the distal end site of the needle body 1, and the raised part 4, 5 is provided to each of the ridge parts 2, 3.

A suture needle according to a ninth aspect is the suture needle according to the sixth aspect, characterized in that a rectilinear ridge part 2, 3 extending in the lengthwise direction of the needle body 1 is provided to the left and right side parts of the distal end site of the needle body 1, and the raised part 4, 5 is provided to each of the ridge parts 2, 3.

A suture needle according to a tenth aspect is the suture needle according to the seventh aspect, characterized in that a rectilinear ridge part 2, 3 extending in the lengthwise direction of the needle body 1 is provided to the left and right side parts of the distal end site of the needle body 1, and the raised part 4, 5 is provided to each of the ridge parts 2, 3.

A suture needle according to an eleventh aspect is the suture needle according to any of the first to third aspects, characterized in that the needle body 1 is formed to be a concavely curved shape from the distal end to a proximal end.

A suture needle according to a twelfth aspect is the suture needle according to the fourth aspect, characterized in that the needle body 1 is formed to be a concavely curved shape from the distal end to a proximal end.

A suture needle according to a thirteenth aspect is the suture needle according to the fifth or sixth aspect, characterized in that the needle body 1 is formed to be a concavely curved shape from the distal end to a proximal end.

A suture needle according to a fourteenth aspect is the suture needle according to the seventh aspect, characterized in that the needle body 1 is formed to be a concavely curved shape from the distal end to a proximal end.

A suture needle according to a fifteenth aspect is the suture needle according to the eighth or ninth aspect, characterized in that the needle body 1 is formed to be a concavely curved shape from the distal end to a proximal end.

A suture needle according to a sixteenth aspect is the suture needle according to the tenth aspect, characterized in that the needle body 1 is formed to be a concavely curved shape from the distal end to a proximal end.

### ADVANTAGEOUS EFFECTS OF THE INVENTION

The present invention, having been configured as described above, therefore serves as an innovative suture needle which exhibits unprecedented effects, such as, e.g., having the greatest possible reduction in insertion resistance when inserted into body tissue, being less likely to stress the body tissue, and allowing for the performance of favorable suturing.

### BRIEF DESCRIPTION OF THE DRAWINGS

FIG. 1 is a perspective view depicting a first embodiment;
FIG. 2 is a plan view of the essential parts according to the first embodiment;
FIG. 3 is a cross-sectional view along line A-A in FIG. 2;
FIG. 4 is a cross-sectional view along line B-B in FIG. 2;
FIG. 5 is bottom view of the essential parts according to the first embodiment;
FIG. 6 is a side view of the essential parts according to the first embodiment;
FIG. 7 is a drawing for describing a method for producing the first embodiment;
FIG. 8 is a drawing for describing the method for producing the first embodiment;
FIG. 9 is a drawing for describing the method for producing the first embodiment;
FIG. 10 is a drawing for describing the method for producing the first embodiment;
FIG. 11 is a drawing for describing the method for producing the first embodiment;
FIG. 12 is a plan view of the essential parts according to a first test body;
FIG. 13 is a plan view of the essential parts according to a second test body;
FIG. 14 is a drawing depicting the results from an insertion performance comparison test for the first test body and the second test body; and
FIG. 15 is a side view of the essential parts according to the second embodiment.

### BEST MODE FOR CARRYING OUT THE INVENTION

Preferred modes for carrying out the present invention shall now be described in brief, with a depiction of the effects of the present invention, on the basis of the accompanying drawings.

When an insertion is to be made in body tissue, a sharp incision will be made into the body tissue by small blades 6, 7 composed of edge parts of an uneven part provided to left and right side parts, and the insertion resistance at the time of insertion into the body tissue is reduced as much as possible. This matter has already been confirmed by an insertion performance comparison test, described below.

Consequently, the insertion resistance at the time of initial insertion into the body tissue is reduced as much as possible, which is the key factor by which subsequent insertion at a site on the proximal end side will also be performed unencumbered. The body tissue will consequently be less likely to be stressed, and favorable suturing work will be performed.

### FIRST EMBODIMENT

A specific first embodiment of the present invention shall now be described on the basis of the accompanying drawings.

The present embodiment is a suture needle to be used in surgical procedures.

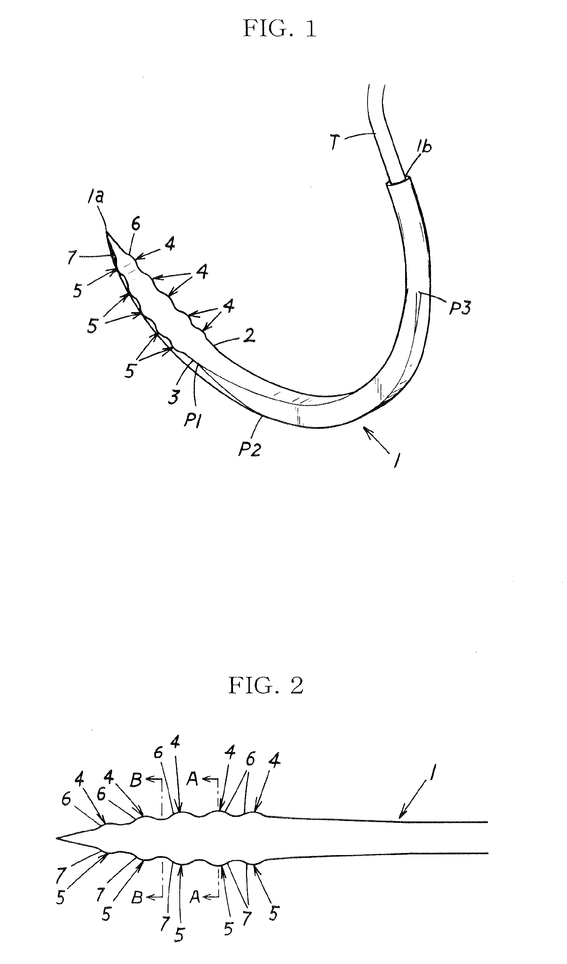

Specifically, a needle body 1 is a rectilinear rod-shaped member made of an appropriate metal that has been formed into a concave curve, as depicted in FIG. 1, and has a distal end to which is provided a sharp part 1a, and a proximal end to which is provided an interlocking part 1b (a hole part) into which a suture thread T is engagingly inserted and interlocked, the interlocking part being shaped to have a circular cross-section.

The total length of the needle body 1 (the length of the rectilinear rod-shaped member) is about 13 mm, and is formed into a concave curve after the formation of raised parts 4, 5 and of polygonal shaped cross-section parts, described below.

The needle body 1 has polygonal shaped cross-section parts which are formed as depicted in FIG. 1.

The present embodiment is configured such that polygonal shaped cross-section parts having different shapes are provided in series in the lengthwise direction of the needle body 1. Specifically, using as a reference a flat surface of the needle body 1 serving as an upper surface thereof (and as an inner surface thereof after having been formed into a concave curve), the flat surface being formed from the distal end of the needle body to a predetermined position P3 toward the proximal end, then each of the polygonal shaped cross-section parts is formed thereon and the polygonal shaped cross-section parts are thus constituted of: a triangular cross-section part provided from the distal end of the needle body 1 until a predetermined position P1 toward the proximal end thereof (being a position about 3 mm from the distal end); a pentagonal cross-section part (a base shape) provided to the proximal end side of the triangular cross-section part (from the predetermined position P1 toward the proximal end until a predetermined position P2 toward the proximal end (at a position about 4.5 mm from the distal end)); a tetragonal cross-section part provided to the proximal end side of the pentagonal cross-section part (from the predetermined position P2 toward the proximal end until a predetermined position P3 toward the proximal end (at a position about 10.5 mm from the distal end)); and a circular cross-section part provided to the proximal end side of the tetragonal cross-section part (from the predetermined position P3 toward the proximal end, until the proximal end).

From this configuration, angular edges 2, 3 (ridge parts), which are provided extending in the lengthwise direction of the needle body 1, have a predetermined length, and are sharp, are provided on an upper surface side of the left and right side parts of the needle body 1, from the distal end thereof until the predetermined position P3 toward the proximal end thereof.

Surfaces between the angular edges on these polygonal cross-section parts (the triangular cross-section shaped part, the pentagonal cross-section shaped part, and the tetragonal cross-section shaped part) may be formed to be concavely-shaped surfaces; in actual practice, a recessed groove part 8, described below, is provided to left and right side surface parts of the triangular cross-section shaped part, and is formed to a shape having a specially shaped triangular cross-section.

The uneven part is provided to the left and right side parts of the distal end site of the needle body 1, and the edge parts of the uneven part are configured as the small blades 6, 7.

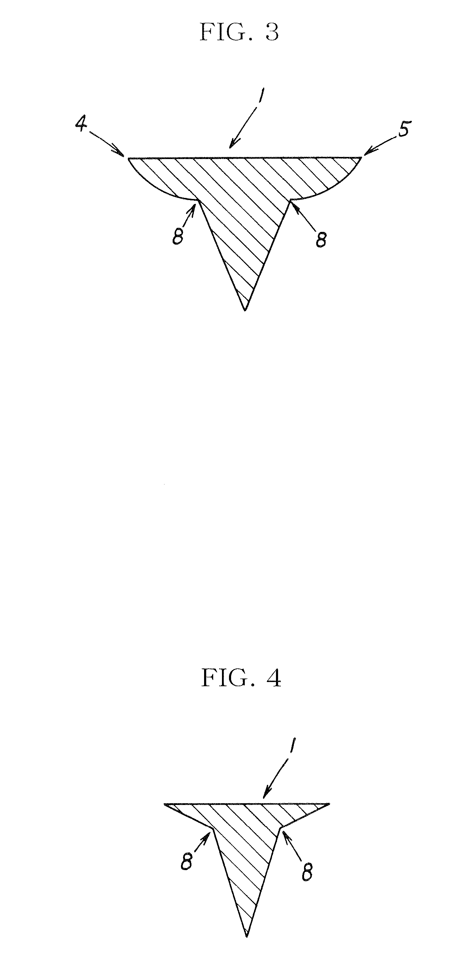

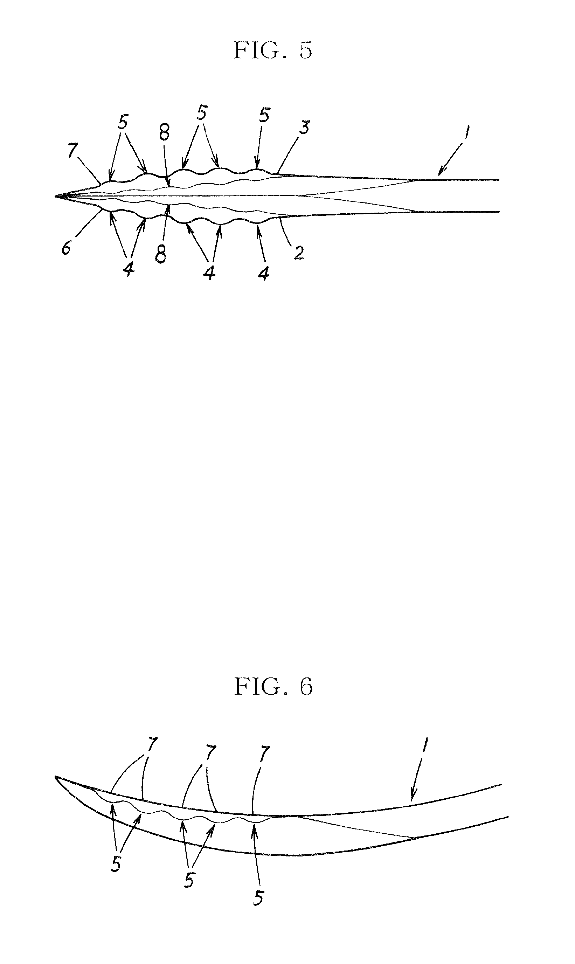

Specifically, with respect to the small blades 6, 7, as depicted in FIGS. 1 to 6, the plurality of raised parts 4, 5 are provided to each of the ridge parts 2, 3 (the angular edges) formed on the left and right side parts of the distal end site (the triangular cross-section shaped part) of the needle body 1, the edge parts of each of the raised parts 4, 5 being configured as the small blades 6, 7; consequently, the small blades 6, 7 are a plurality provided in parallel in the lengthwise direction of the needle body 1.

In other words, the uneven part is formed on the left and right side parts of the needle body 1, and the edge parts of the distal end side of the needle body 1 at each of the raised parts 4, 5 are configured as the small blades 6, 7.

What follows is a description of a method for producing the small blades 6, 7 provided to the left and right side parts of the distal end site of the needle body 1.

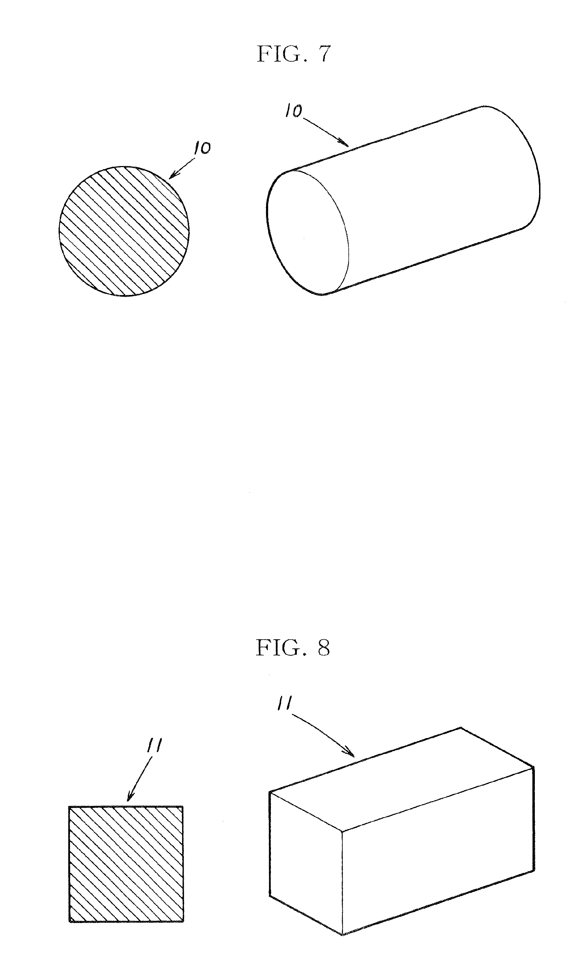

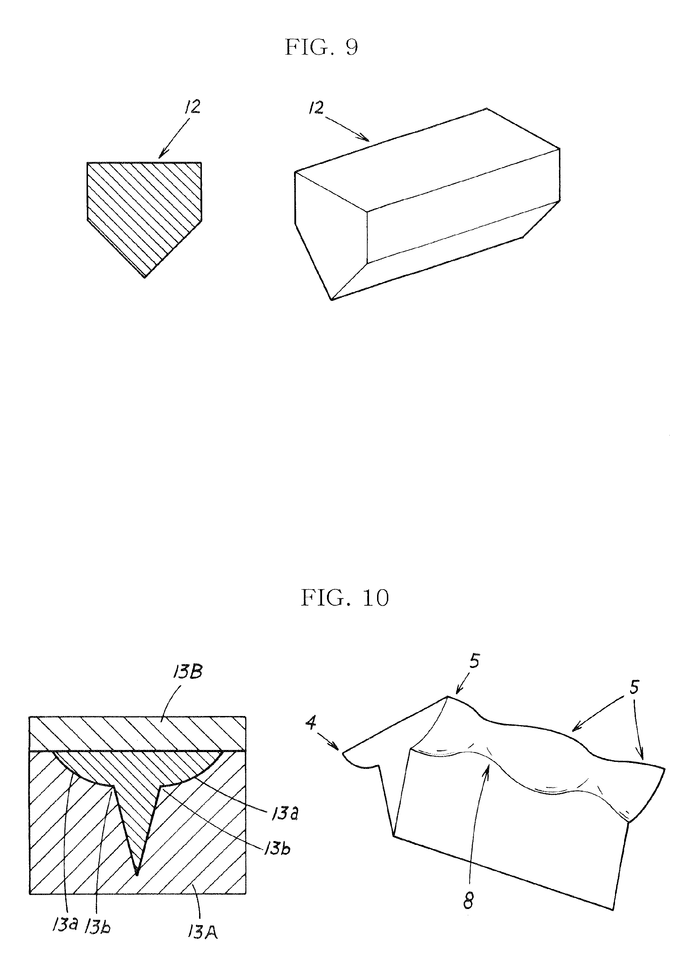

First, a site 10 of the rectilinear rod-shaped member, the site shaped to have a circular cross-section, is pressed to form a site 11 shaped to have a tetragonal cross-section, as depicted in FIGS. 7 and 8, following which the site 11 shaped to have a tetragonal cross-section is cut to form a site 12 shaped to have a pentagonal cross-section, as depicted in FIG. 9.

Then, the site 12 shaped to have the pentagonal cross-section is pressed with upper and lower metallic molds 13A, 13B to provide the recessed groove part 8 to the left and right side parts and to provide the raised parts 4, 5 to an upper position of the left and right side parts, as depicted in FIG. 10. The recessed groove part 8 is formed using an angled raised part 13b formed of a bulge on left and right sites of the lower metallic mold 13A, and each of the raised parts 4, 5 is formed using an arcuate recessed part 13a provided as a recession in the lower metallic mold 13A. The arcuate recessed parts 13a are provided as a plurality in parallel in the lengthwise direction of the lower metallic mold 13A, and arc-shaped raised parts (not shown) provided between the arcuate recessed parts 13a are used to form recessed parts (which also include recessed parts for constituting the recessed groove part 8) between each of the raised parts 4, 5 provided to the left and right side parts of the needle body 1.

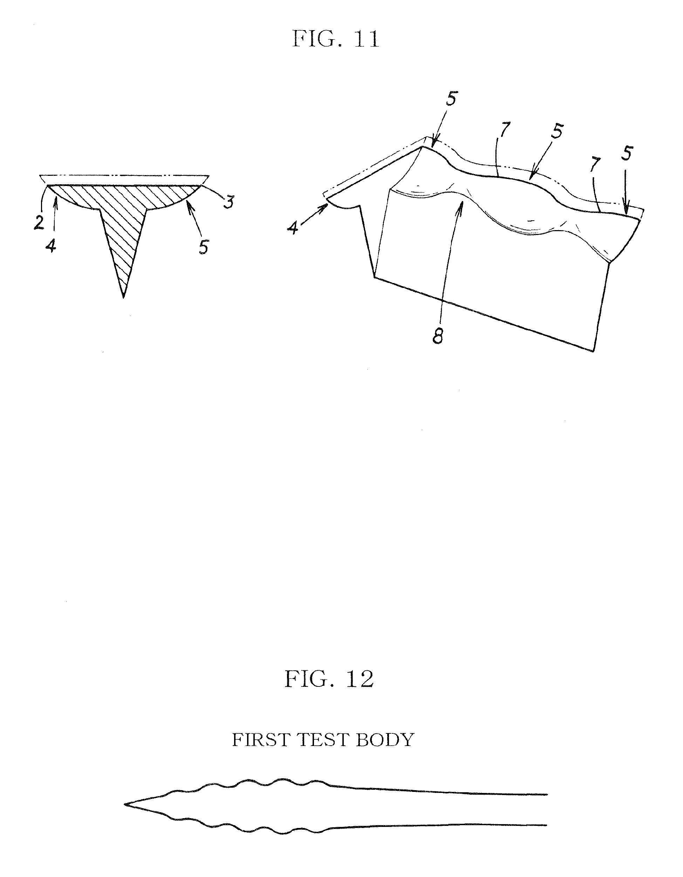

Then, an upper portion of the site 12 having a triangular cross-section is cut, as depicted in FIG. 11, whereupon the ridge parts 2, 3 (the angular edges), which are provided extending in the lengthwise direction of the site 12 having a triangular cross-section, which are of a predetermined length, and which are rectilinear, are provided to the site 12 having a triangular cross-section; the raised parts 4, 5 are provided to each of the ridge parts 2, 3, and the ridge parts of each of the raised parts 4, 5 are used to form the small blades 6, 7.

In an alternative configuration of the small blades 6, 7, a plurality of recessed parts is provided to the left and right side parts of the distal end site of the needle body 1, spaces being interposed therebetween, and edge parts of portions between these recessed parts are configured as the small blades 6, 7.

In the present embodiment, the left and right side parts of the distal end site of the needle body 1 have the rectilinear recessed groove part 8 provided in the lengthwise direction of the needle body 1, as described above. However, the recessed groove parts 8 may also be provided to each as a plurality.

The recessed groove parts 8 reduce the surface area of contact with the body tissue when, for example, the needle body 1, having been inserted into the body tissue, is pulled out, wherefore providing the recessed groove parts 8 reduces as much as possible the insertion resistance and pull-out resistance with respect to the body tissue.

Because the present embodiment has the aforedescribed configuration, the small blades 6, 7 of the raised parts 4, 5 allow for a sharp and punctuated incision into the body tissue upon, for example, insertion into the body tissue, and thus the result of providing a plurality of raised parts 4, 5 to the left and right side parts of the distal end site of the needle main boy 1 and of providing the plurality of small blades 6, 7 thereto is that the insertion resistance upon insertion into the body tissue will be reduced as much as possible. Specifically, the raised parts 4, 5, by each being in a plurality and by each having the plurality of small blades 6, 7, cause the left and right side parts of the distal end part of the needle body 1 to make the incision in a punctuated manner, rather than continuously constant, upon insertion into the body tissue, whereby favorable insertion is achieved.

The present applicants have confirmed, through testing, that, as described above, the insertion resistance is reduced as much as possible.

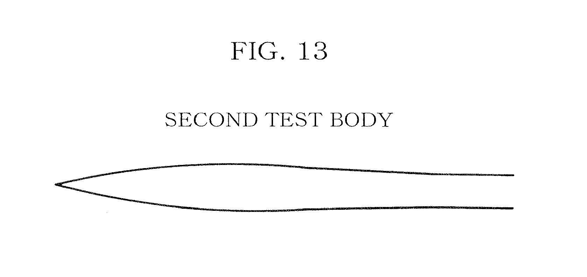

Specifically, there were prepared a first test body structured according to the present embodiment (a structure in which the raised parts 4, 5 were provided to the left and right side parts of the distal end site of the needle body 1), as depicted in FIG. 12, and a second test body in which the raised parts 4, 5 were not imparted to the left and right side parts of the distal end site of the needle body 1, as depicted in FIG. 13; both were subjected to an insertion performance comparison test.

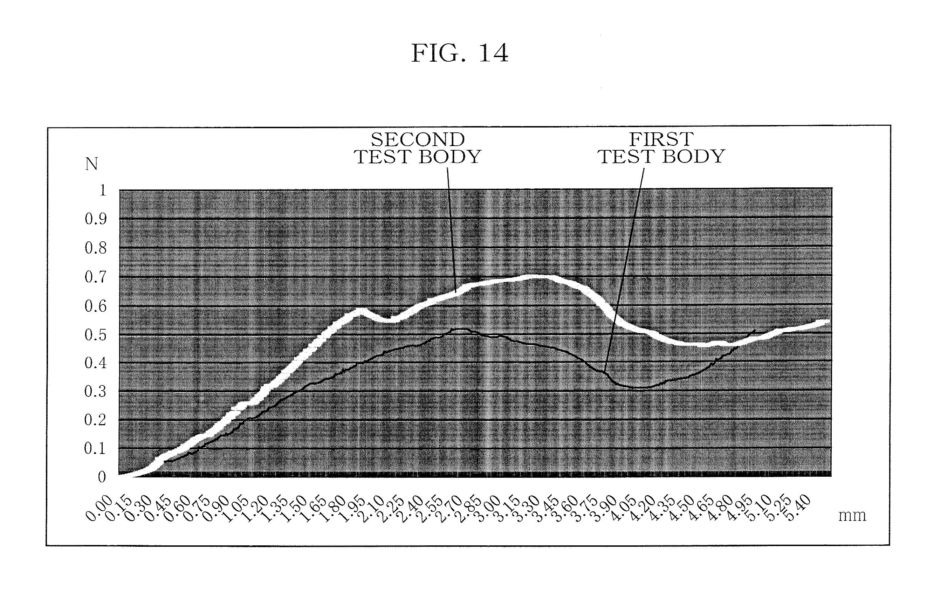

This insertion performance comparison test is conducted to measure and compare the frictional resistance values generated when the first test body and the second test body were inserted into an identical insertion-receiving member at the same pressure; the results thereof are represented in the graph depicted in FIG. 14. The horizontal axis of the graph is the length (in millimeters) from the distal ends of the first test body and the second test body, and the vertical axis is the frictional resistance value (in newtons) generated upon insertion into the insertion-receiving member.

The black line in the graph represents the first test body, and the white line represents the second test body; it can be seen from the graph that, compared to the second test body, the first test body had lower frictional resistance values at a position about 0.6 mm from the distal end of the needle body 1, where the initial raised parts 4, 5 are provided, up to a position about 2.5 mm from the distal end of the needle body 1, where the final raised parts 4, 5 are provided. Presumably because of the low insertion resistance resulting from the passing of the positions where the raised parts 4, 5 are provided, the frictional resistance value at subsequent sites is also low.

In view of the above circumstances, this test has shown that providing the raised parts 4, 5 (the small blades 6, 7) to the left and right side parts of the distal end site of the needle body 1 is extremely effective.

Therefore, according to the present embodiment, the insertion resistance at the left and right side parts, being the distal end site of the needle body 1 where resistance is applied at the time of initial insertion into the body tissue, is in particular reduced as much as possible, which is the key factor by which subsequent insertion at a site toward the proximal end of the needle body 1 will also be performed unencumbered, and the body tissue will consequently be less likely to be stressed, and favorable suturing work will be performed.

Also, since, in the present embodiment, the left and right side parts of the distal end site of the needle body 1 have the recessed groove parts 8 provided in the lengthwise direction of the needle body 1, the presence of the recessed groove parts 8 also reduces the surface area of contact with the body tissue, wherefore insertion resistance with respect to the body tissue is reduced as much as possible.

In the present embodiment, in which the polygonal cross-section parts are formed on the needle body 1, the angular edges on these polygonal cross-section parts allow for a sharp incision to be made into the body tissue, and allow for the greatest possible reduction in insertion resistance upon insertion into the body tissue.

Since the polygonal cross-section parts according to the present embodiment are configured such that the polygonal cross-section parts having different shapes are provided in series in the lengthwise direction of the needle body 1, there occur changes in shape throughout the insertion process, whereby an effect on the incision will be created and whereby the insertion resistance upon insertion into the body tissue will be reduced as much as possible.

Specifically, since, in the present embodiment, the polygonal cross-section shaped parts are constituted of the triangular cross-section shaped part provided to the distal end side of the needle body 1 as well as of the pentagonal cross-section shaped part and tetragonal cross-section shaped part provided closer to the proximal end than the triangular cross-section shaped part, there occur changes in shape in that the number of angles changes throughout the insertion process, whereby an effect on the incision will be created and whereby the insertion resistance upon insertion into the body tissue will be reduced as much as possible.

### SECOND EMBODIMENT

A specific second embodiment of the present invention shall now be described on the basis of the accompanying drawings.

In the present embodiment, in addition to the provision of the raised parts 4, 5 and the small blades 6, 7 to the left and right side parts of the needle body 1, the distal end part of the needle body 1 is provided with the uneven part at an outer surface part (lower surface part) of the triangular cross-section shaped part, and the edge parts of this uneven part are configured as small blades 15.

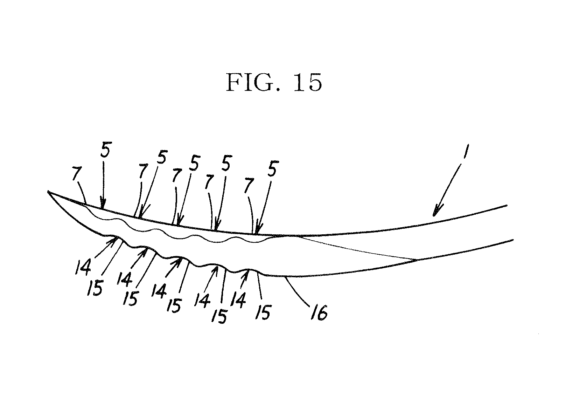

Specifically, a plurality of recessed parts 14 are provided, in the axial direction of the needle body 1, to a ridge part 16 (an angular edge) formed on the outer surface part of the distal end part (the triangular cross-section shaped part) of the needle body 1, as depicted in FIG. 15, and an edge part of the recessed parts 14 is configured as the small blades 15, the small blades 15 protruding outward and provided as a plurality in series in the lengthwise direction of the needle body 1.

The recessed parts 14 may be formed by pressing, similarly with respect to the aforedescribed raised parts 4, 5 of the left and right side parts, or may be formed by cutting.

The present invention is not to be limited to the first and second embodiments; the specification configuration of each of the constituent elements can be designed as appropriate.

## Claims

1. A suture needle, **characterized in that** an uneven part is provided to left and right side parts of a distal end site of a needle body, and an edge part of the uneven part is configured as a small blade.

2. The suture needle according to Claim 1, **characterized in that** the small blade is provided to an edge part of a raised part of the uneven part.

3. The suture needle according to Claim 2, **characterized in that** the raised part is a provided in a plurality in a lengthwise direction of the needle body.

4. The suture needle according to any of Claims 1 to 3, **characterized in that** the distal end site of the needle body is configured to have a triangular shape in cross-section, and the uneven part is provided to each of left and right side edges of the distal end site having a triangular shape in cross-section.

5. A suture needle, **characterized in that** a raised part is provided to left and right side parts of a distal end site of a needle body, and an edge part of each of the raised parts on the left and right is configured as a small blade.

6. The suture needle according to Claim 5, **characterized in that** a plurality of each of the raised parts on the left and right is provided.

7. The suture needle according to Claim 5 or 6, **characterized in that** a plurality of each of the raised parts on the left and right is provided in series along a lengthwise direction of the needle body.

8. The suture needle according to Claim 5, **characterized in that** a rectilinear ridge part extending in the lengthwise direction of the needle body is provided to the left and right side parts of the distal end site of the needle body, and the raised part is provided to each of the ridge parts.

9. The suture needle according to Claim 6, **characterized in that** a rectilinear ridge part extending in the lengthwise direction of the needle body is provided to the left and right side parts of the distal end site of the needle body, and the raised part is provided to each of the ridge parts.

10. The suture needle according to Claim 7, **characterized in that** a rectilinear ridge part extending in the lengthwise direction of the needle body is provided to the left and right side parts of the distal end site of the needle body, and the raised part is provided to each of the ridge parts.

11. The suture needle according to any of Claims 1 to 3, **characterized in that** the needle body is formed to be a concavely curved shape from the distal end to a proximal end.

12. The suture needle according to Claim 4, **characterized in that** the needle body is formed to be a concavely curved shape from the distal end to a proximal end.

13. The suture needle according to Claim 5 or 6, **characterized in that** the needle body is formed to be a concavely curved shape from the distal end to a proximal end.

14. The suture needle according to Claim 7, **characterized in that** the needle body is formed to be a concavely curved shape from the distal end to a proximal end.

15. The suture needle according to Claim 8 or 9, **characterized in that** the needle body is formed to be a concavely curved shape from the distal end to a proximal end.

16. The suture needle according to Claim 10, **characterized in that** the needle body is formed to be a concavely curved shape from the distal end to a proximal end.
